# EUROPEAN PATENT SPECIFICATION

(11) **EP 4 115 925 B1**
(45) Date of publication and mention of the grant of the patent: **13.08.2025**
(21) Application number: 21771625.7
(22) Date of filing: 08.03.2021
(51) Int. Cl.: A61M 5/145, A61M 39/04, A61M 39/20, A61M 5/31

(54) **SYRINGE ASSEMBLY AND DRUG SOLUTION ADMINISTRATION DEVICE**
SPRITZENANORDNUNG UND VORRICHTUNG ZUR VERABREICHUNG EINER ARZNEIMITTELLÖSUNG
ENSEMBLE SERINGUE ET DISPOSITIF D'ADMINISTRATION DE SOLUTION MÉDICAMENTEUSE

(30) Priority: 17.03.2020 JP 2020045900
(43) Date of publication of application: 11.01.2023
(73) Proprietor: TERUMO Kabushiki Kaisha, Tokyo 151-0072 (JP)
(72) Inventor: OGAWA, Junichi, Nakakoma-gun, Yamanashi 409-3853 (JP)
(74) Representative: Casalonga
(86) International application number: PCT/JP2021/008872
(87) International publication number: WO 2021/187182

(56) References cited:
- EP-A1- 2 823 842
- WO-A1-2019/182031
- WO-A1-2019/182031
- DE-A1- 102011 111 552
- JP-A- 2016 508 404
- US-A- 3 989 044
- US-A1- 2007 156 100
- US-A1- 2016 184 529

## Description

### Technical Field

The present invention relates to a syringe assembly sealing a liquid medicine according to the preamble of claim 1 and a liquid-medicine administration device.

### Background Art

Conventionally, there is proposed a liquid-medicine administration device of a syringe pump type in which a liquid medicine charged in a cylindrical body is administered into a living body under pressing action of a plunger (WO 2019/182031 A). The liquid-medicine administration device includes a syringe assembly filled with a liquid medicine. A syringe assembly includes a syringe having a distal nozzle portion, a cap fixed to the distal nozzle portion, and a seal member arranged between the cap and the distal nozzle portion and made of an elastic body that seals an opening of the distal nozzle portion.
US 2016/184529 A1 relates to a pre-filled syringe and a structure for preventing the loosening of the cap.
WO 2019/182031 A1 relates to a cap, a syringe assembly, and a method for manufacturing the same and further provides a cap that does not easily come off from the tip nozzle portion of the syringe.
US 3 989 044 A relates to a prefilled syringe having a barrel to hold a liquid medium and a needle hub assembly whereby the integrity of the liquid medium remains static until the assembly is activated and the stopper pierced.
DE 10 2011 111552 A1 relates to a syringe with a safety cap and the closure cap that are formed in one piece.

### Summary of Invention

In an assembly step of the syringe assembly, there is a problem that the compression of the seal member becomes non-uniform in the circumferential direction, an internal pressure (pressure limit) at which liquid leakage occurs varies, and the syringe assembly having an extremely low pressure limit is formed.

In the cap, a step of aligning an axis of the cap and an axis of the syringe coaxially and fitting the cap toward the distal nozzle portion is performed by a mounting device. Meanwhile, it has been found that the axis of the cap is sometimes inclined with respect to the axis of the syringe when the cap is assembled, a crushing load on the seal member by the distal nozzle portion varies to cause a variation in the pressure limit.

Therefore, an object of one embodiment is to provide a syringe assembly and a liquid-medicine administration device capable of suppressing a variation in a pressure limit of a seal member.

An aspect of the following disclosure relates to a syringe assembly including: a syringe having a distal nozzle portion; a cap fixed to the distal nozzle portion; and a seal member arranged between the cap and the distal nozzle portion and made of an elastic body that seals the distal nozzle portion, the cap having a pressing surface that is in surface contact with a distal end of the seal member and presses the seal member to a proximal side, the distal nozzle portion having a sealing surface, which opposes the pressing surface and is in surface contact with a proximal end of the seal member, at a distal end and a distal opening which is formed on an inner peripheral side of the sealing surface and communicates with a lumen of the syringe, the seal member closing the distal opening and being pressed and held in an axial direction in a pressing space over a whole region of an opposing portion between the pressing surface and the sealing surface, and a pressing space between the pressing surface and the sealing surface being narrowest on an inner peripheral side, wherein the sealing surface is provided with a ring-shaped protrusion formed in an annular shape along an inner peripheral side of the sealing surface and protruding toward the pressing surface.

Another aspect relates to a liquid-medicine administration device including: the syringe assembly according to the above aspect; a gasket arranged to be slidable inside the syringe; a plunger assembly capable of pressing the gasket in a distal direction, and a drive mechanism driving the plunger assembly.

According to the syringe assembly and the liquid-medicine administration device of the above aspects, the crushing load on the seal member by the distal nozzle portion is made uniform, and the variation in the pressure limit is suppressed.

### Brief Description of Drawings

Fig. 1 is a schematic diagram of a liquid-medicine administration device including a syringe assembly according to a first embodiment.
Fig. 2 is a perspective view of the liquid-medicine administration device of Fig. 1 from which a housing has been removed.
Fig. 3 is a cross-sectional view of the distal side of the syringe assembly of Fig. 2.
Fig. 4 is a cross-sectional view of the distal side of a syringe assembly according to a comparative example.
Fig. 5 is a cross-sectional view illustrating action of the syringe assembly of Fig. 3.
Fig. 6 is a graph illustrating a measurement result of a pressure limit of the syringe assembly according to Experimental Example 1 (the comparative example).
Fig. 7 is a graph illustrating a measurement result of a pressure limit of the syringe assembly according to Experimental Example 2 (the first embodiment).
Fig. 8 is a cross-sectional view of the distal side of a syringe assembly according to an illustrative example not falling under the scope of the claims.
Fig. 9 is a cross-sectional view illustrating action of the syringe assembly of Fig. 8.
Fig. 10 is a cross-sectional view of a distal side of a syringe assembly according to an illustrative example not falling under the scope of the claims.
Fig. 11 is a cross-sectional view illustrating action of the syringe assembly of Fig. 10.
Fig. 12 is a cross-sectional view of a distal side of a syringe assembly according to an illustrative example not falling under the scope of the claims.
Fig. 13 is a cross-sectional view illustrating action of the syringe assembly of Fig. 12.

### Description of Embodiments

Hereinafter, preferred embodiments regarding syringe assemblies 12A, 12B, 12C, and 12D and a liquid-medicine administration device 10 will be described in detail with reference to the accompanying drawings. Incidentally, only the main parts will be illustrated in second to fourth embodiments, and the same or similar configurations as those of the first embodiment will be denoted by the same reference signs, and the detailed description thereof will be omitted.

### [First Embodiment]

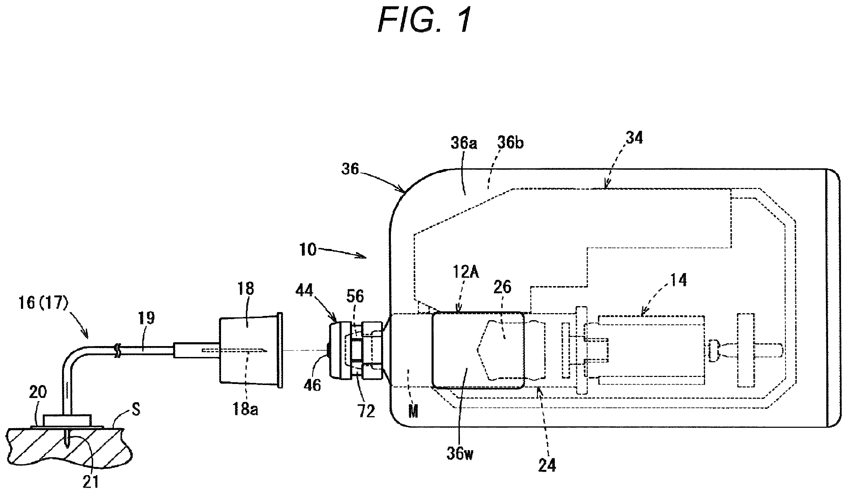

The liquid-medicine administration device 10 illustrated in Fig. 1 is used to administer a liquid medicine M into a living body. The liquid-medicine administration device 10 continuously administers the liquid medicine M charged in the syringe assembly 12A into the living body under pressing action of a plunger assembly 14 for a relatively long time (for example, about several minutes to several hours). The liquid-medicine administration device 10 may intermittently administer the liquid medicine M into the living body. Examples of the liquid medicine M include a protein preparation, a narcotic analgesic, a diuretic, and the like.

As illustrated in Fig. 1, when the liquid-medicine administration device 10 is used, for example, a patch-type needle-attached tube 17 is connected as an administration tool 16. The liquid medicine M discharged from the syringe assembly 12A is injected into the body of a patient via the needle-attached tube 17. The needle-attached tube 17 includes: a connector 18 that can be connected to a distal nozzle portion 56 of the syringe assembly 12A; a liquid supply tube 19 having one end connected to the connector 18 and having flexibility; a patch portion 20 that is connected to the other end of the liquid supply tube 19 and can be stuck to a skin S; and a puncture needle 21 protruding from the patch portion 20. The puncture needle 21 substantially perpendicularly punctures the skin S. Incidentally, the puncture needle 21 may be one that obliquely punctures the skin S.

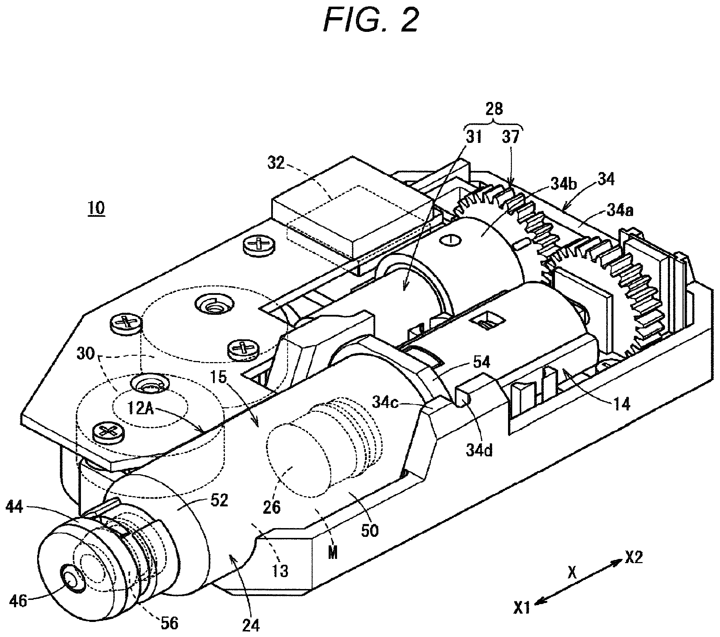

As illustrated in Fig. 1 or 2, the liquid-medicine administration device 10 includes: the syringe assembly 12A having a syringe 24 filled with the liquid medicine M; a gasket 26 arranged to be slidable inside the syringe 24; the plunger assembly 14 that is stretchable in the axial direction (arrow X direction) and can press the gasket 26 in the distal direction (arrow X1 direction); a drive mechanism 28 that drives the plunger assembly 14; a battery 30 that supplies electric power necessary for the operation of the liquid-medicine administration device 10; a control unit 32 that controls the drive mechanism 28; a chassis structure 34 that supports the syringe assembly 12A, the plunger assembly 14, and the drive mechanism 28; and a housing 36 that houses these.

As illustrated in Fig. 2, the syringe assembly 12A includes: the syringe 24 having the distal nozzle portion 56; a cap 44 mounted on the distal nozzle portion 56 of the syringe 24; and a seal member 46 arranged between the distal nozzle portion 56 and the cap 44.

The syringe 24 is formed in a hollow cylindrical shape. Specifically, the syringe 24 includes a barrel portion 50 having a lumen 13 that can be filled with the liquid medicine M, and a flange portion 54 protruding outward from an outer peripheral surface of the barrel portion 50. The inside of the syringe 24 is filled with the liquid medicine M in advance. The syringe 24 may be made of a transparent material.

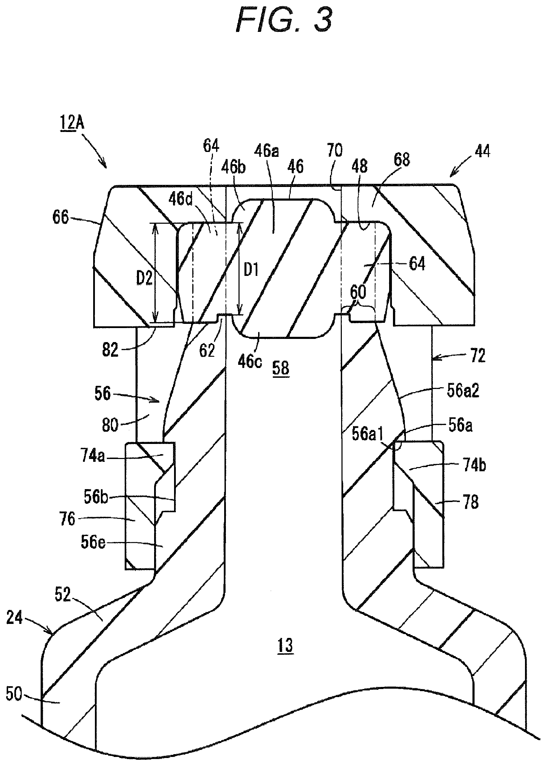

As illustrated in Fig. 3, a distal portion of the distal nozzle portion 56 is provided with an engagement projecting portion 56a. The engagement projecting portion 56a is formed in an annular shape that protrudes radially outward and extends to make a round in the circumferential direction. The engagement projecting portion 56a includes: a locking surface 56a1 on which claw portions 74a and 74b, which will be described later, of the cap 44 are locked; and an inclined surface 56a2 which is formed on the distal side of the locking surface 56al and reduced in diameter in the distal direction. The locking surface 56al is a flat surface perpendicular to an axis of the distal nozzle portion 56. On an outer peripheral surface of the distal nozzle portion 56, an annular groove 56b that is recessed radially inward is formed on the proximal side of the engagement projecting portion 56a.

An anti-rattling projecting portion 56e that prevents rattling of the cap 44 is provided on the outer periphery of a proximal portion of the distal nozzle portion 56. The anti-rattling projecting portion 56e is formed in an annular shape that bulges radially outward and extends to make a round in the circumferential direction. An outer peripheral surface of the anti-rattling projecting portion 56e extends along the axis of the distal nozzle portion 56. A proximal end of the anti-rattling projecting portion 56e is connected to a distal end of the shoulder portion 52.

A sealing surface 60 perpendicular to the axial direction is formed at a distal end of the distal nozzle portion 56. The sealing surface 60 is formed in a circular ring shape on the outer peripheral side of a distal opening 58. Since the substantially entire radial region of the sealing surface 60 is in surface contact with the seal member 46, high sealing performance (pressure resistance) is exhibited, and liquid leakage can be prevented even if a relatively high pressure of the lumen 13 acts. The sealing surface 60 can have an inner diameter set to about 0.5 to 5.0 mm, and an outer diameter set to about 2.0 to 9.0 mm.

A ring-shaped protrusion 62 protruding toward the axially distal side (a pressing surface 48) is formed on the inner peripheral side of the sealing surface 60. The ring-shaped protrusion 62 is formed in an annular shape along an inner peripheral portion of the sealing surface 60. A protruding height of the ring-shaped protrusion 62 is formed to such an extent as not to hinder the surface contact between an outer peripheral portion 46d of the seal member 46 and the sealing surface 60. The ring-shaped protrusion 62 prevents movement of a wall of the seal member 46 from moving when the cap 44 is mounted. As dimensions of the ring-shaped protrusion 62 exhibiting such a function, for example, a protruding height in the axial direction can be set to about 0.05 to 1.5 mm and a dimension in the radial direction (width direction) can be set to about 0.05 to 1.5 mm.

The seal member 46 is punctured by a needle 18a provided in the connector 18 when the connector 18 illustrated in Fig. 1 is connected to the distal nozzle portion 56. The seal member 46 is made of an elastic resin material such as a rubber material or an elastomer material formed in a plate shape. The seal member 46 is fixed to the distal nozzle portion 56 of the syringe assembly 12A by the cap 44 to seal the distal opening 58 of the distal nozzle portion 56. The seal member 46 is held in a state of being elastically compressed in the axial direction between the sealing surface 60 of the distal nozzle portion 56 and the pressing surface 48 of the cap 44. The seal member 46 is formed in a disc shape.

The seal member 46 includes: a seal body portion 46a forming a central portion in the thickness direction; a distal projecting portion 46b protruding in the distal direction from the distal side of the seal body portion 46a; and a proximal projecting portion 46c protruding in the proximal direction from the proximal side of the seal body portion 46a. The distal projecting portion 46b slightly protrudes in the distal direction from the pressing surface 48 of the cap 44. A surface on the distal side of the distal projecting portion 46b may be located at the same axial position as the pressing surface 48 of the cap 44 or on the proximal side of the pressing surface 48.

The outer peripheral portion 46d of the distal projecting portion 46b and a proximal projecting portion 46c of the seal member 46 is arranged in a pressing space 64 formed between the sealing surface 60 of the distal nozzle portion 56 and the pressing surface 48 of the cap 44. In the pressing space 64, a proximal end of the outer peripheral portion 46d of the seal member 46 is in surface contact and close contact with substantially the entire radial region of the sealing surface 60, thereby exhibiting the pressure resistance. In addition, the outer peripheral portion 46d of the seal member 46 is in surface contact with the pressing surface 48 of the cap 44 on the distal side. Then, the outer peripheral portion 46d is pressed in the axial direction by the pressing surface 48 and the sealing surface 60.

The pressing space 64 is a space between the sealing surface 60 of the distal nozzle portion 56 and the pressing surface 48 of the cap 44 at a portion opposing the sealing surface, and is formed in a circular ring shape on the outer peripheral side of the distal opening 58 of the distal nozzle portion 56. Since the ring-shaped protrusion 62 is formed along the inner peripheral side of the sealing surface 60 in the present embodiment, a width D1 on the inner peripheral side is narrower than a width D2 on the outer peripheral side regarding a width D (gap) of the pressing space 64 in the axial direction. That is, the width D of the pressing space 64 in the axial direction is narrowest on the inner peripheral side.

The cap 44 includes a base portion 66 provided on the distal side of the cap 44, and a mounting portion 72 which has a cylindrical shape, extends from the base portion 66 in the proximal direction along an axis of the cap 44, and covers the outside of the distal nozzle portion 56. The pressing surface 48 that is in surface contact with the seal member 46 is formed on the proximal side of the base portion 66. The pressing surface 48 is formed in a circular ring shape around a through-hole 70 at the center. The through-hole 70 is formed in a central portion of the base portion 66 to penetrate the base portion in the axial direction and expose the distal projecting portion 46b of the seal member 46.

The through-hole 70 is a hole formed in a circular shape when viewed from the axial direction, and is formed coaxially with the cap 44. The through-hole 70 is formed in a distal wall 68 of the base portion 66. The distal projecting portion 46b of the seal member 46 is inserted into the through-hole 70.

The mounting portion 72 includes claw portions 74a and 74b, a first column portion 76, and a second column portion 78. The two claw portions 74a and 74b oppose each other across the central axis of the cap 44, and are formed at positions spaced apart from the base portion 66 to the proximal side. The claw portions 74a and 74b protrude inward from an inner peripheral surface of the mounting portion 72. The cap 44 is mounted to the distal nozzle portion 56 as the two claw portions 74a and 74b engage with the engagement projecting portion 56a of the distal nozzle portion 56. That is, the two claw portions 74a and 74b engage with the proximal end (locking surface 56a1) of the engagement projecting portion 56a of the distal nozzle portion 56, thereby preventing the cap 44 from coming off from the distal nozzle portion 56. Each of the two claw portions 74a and 74b extends in an arc shape in the circumferential direction along the inner peripheral surface of the mounting portion 72.

The first column portion 76 is provided on the outer side of one claw portion 74a in the circumferential direction of the mounting portion 72, and extends to the proximal side along the axis of the cap 44. The second column portion 78 is provided on the outer side of the other claw portion 74b in the circumferential direction of the mounting portion 72, and extends to the proximal side along the axis of the cap 44. Inner peripheral surfaces of the first column portion 76 and the second column portion 78 abut on the anti-rattling projecting portion 56e of the distal nozzle portion 56, thereby preventing the cap 44 from being inclined with respect to the distal nozzle portion 56. The first column portion 76 and the second column portion 78 are integrally connected to the base portion 66 via the side wall portion 80. Two side holes 82 are formed on the distal side of the side wall portion 80. The side holes 82 penetrate the side wall portion 80 in the radial direction.

In Fig. 2, the gasket 26 liquid-tightly seals the proximal side of the lumen 13 of the syringe 24. In an initial state of the liquid-medicine administration device 10, the gasket 26 is located on the distal side of a proximal end of the syringe 24. An outer portion of the gasket 26 is in close contact with an inner peripheral surface of the syringe 24 (barrel portion 50) in a liquid-tight manner. The syringe assembly 12A, the liquid medicine M, and the gasket 26 form a prefilled syringe 15.

The plunger assembly 14 is configured to advance the gasket 26 inside the syringe 24 and push out the liquid medicine M from the syringe assembly 12A. In the initial state of the liquid-medicine administration device 10, the distal side of the plunger assembly 14 is inserted into the proximal side of the syringe 24. The drive mechanism 28 includes: a motor 31 which is driven and controlled under control of the control unit 32 using the battery 30 as a power source; and a drive gear 37 fixed to an output shaft of the motor 31.

The chassis structure 34 is arranged inside the housing 36 (see Fig. 1). The syringe assembly 12A, the drive mechanism 28, and the plunger assembly 14 are fixed to predetermined positions of the chassis structure 34, respectively. The chassis structure 34 includes a chassis body member 34a and a motor holding member 34b that is fixed to the chassis body member 34a and holds the motor 31 against the chassis body member 34a.

The chassis body member 34a has a flange holding portion 34c that protrudes upward and holds the flange portion 54 of the syringe 24. The flange holding portion 34c is provided with a holding groove 34d into which the flange portion 54 is inserted.

In Fig. 1, the housing 36 is a hollow member configured to house the syringe assembly 12A, the gasket 26, the plunger assembly 14, the drive mechanism 28, the battery 30, the control unit 32, and the chassis structure 34 described above. The distal nozzle portion 56 of the syringe assembly 12A protrudes from the housing 36, and the cap 44 is exposed to the outside. A window portion 36w made of a transparent material is provided on an upper surface 36a of the housing 36.

The liquid-medicine administration device 10 can be configured as a patch type that is used by being stuck to the skin S of the patient, for example. In the case of such a patch type, a sheet-shaped sticking portion (adhesive portion) that can be stuck to the skin S is provided on a bottom surface 36b of the housing 36. In the initial state of the liquid-medicine administration device 10, a peelable protective sheet is stuck to a sticking surface of the sticking portion.

Incidentally, the liquid-medicine administration device 10 is provided with a mounting tool, such as a hook and a clip, on the bottom surface 36b of the housing 36 and mounted by a method of being hooked on clothes of the patient (for example, a waist portion of pants or the like).

Next, the action (assembly step) of the syringe assembly 12A will be described with reference to a comparative example.

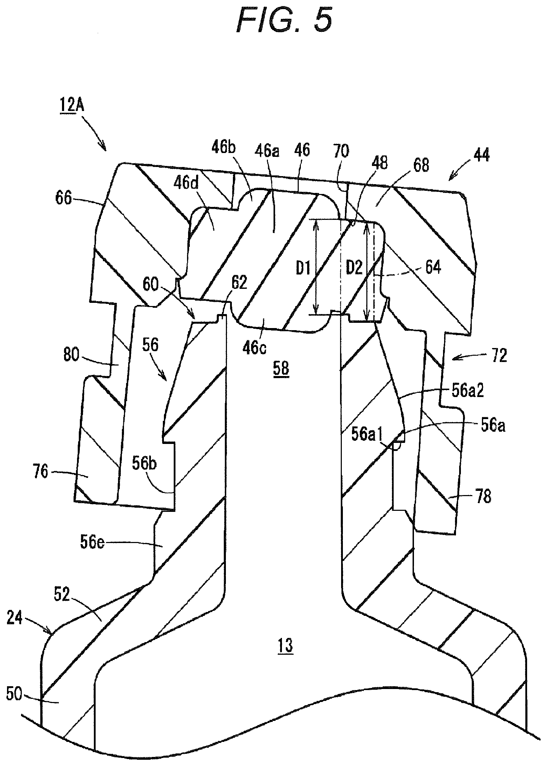

In the assembly step of the syringe assembly 12A, a syringe preparation step, a seal member preparation step, a cap preparation step, a cap assembly step, and a cap mounting step (see Fig. 5) are sequentially performed.

In the syringe preparation step, the syringe 24 including the distal nozzle portion 56 having the engagement projecting portion 56a provided on the outer peripheral surface and the distal opening 58 is prepared.

In the seal member preparation step, the plate-shaped seal member 46 having elasticity is prepared.

In the cap preparation step, the cap 44 including the base portion 66, made of a hard resin material and provided at the distal end, and the cylindrical mounting portion 72, which extends from the base portion 66 in the proximal direction along the axis of the cap 44, is prepared.

In the cap assembly step, the seal member 46 is inserted into the inner peripheral side of the mounting portion 72 of the cap 44, and the seal member 46 is in surface contact with the pressing surface 48. In addition, the distal projecting portion 46b of the seal member 46 is inserted into the through-hole 70.

In the cap mounting step, the distal nozzle portion 56 is inserted into the mounting portion 72 of the cap 44 to which the seal member 46 is attached. Then, the cap 44 is pushed to the proximal side until the two claw portions 74a and 74b of the mounting portion 72 pass over the engagement projecting portion 56a of the distal nozzle portion 56. Then, the distal opening 58 of the distal nozzle portion 56 is sealed with the seal member 46.

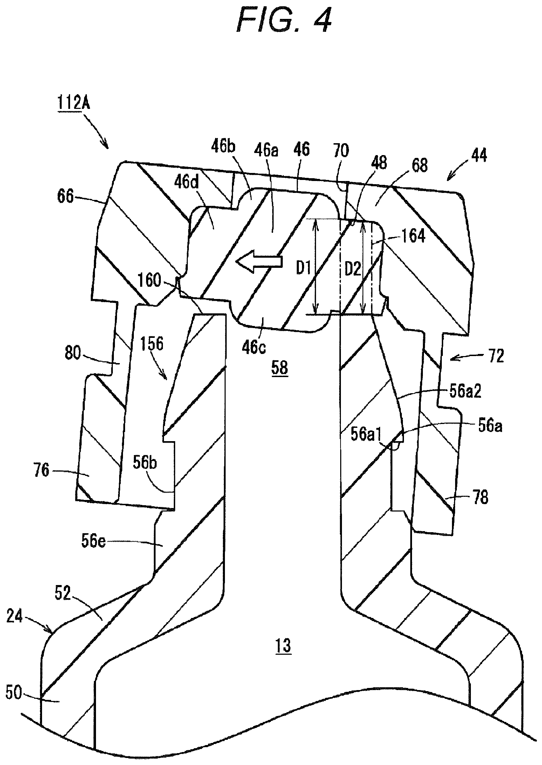

The cap mounting step is performed by press-fitting the cap 44 toward the distal nozzle portion 56 while gripping the cap 44 and the syringe 24 with a chuck of the mounting device. In a state in Fig. 5 where the cap mounting step is completed, the inner peripheral surface of the mounting portion 72 of the cap 44 abuts on the anti-rattling projecting portion 56e of the distal nozzle portion 56, and the cap 44 is assembled without being inclined. However, in the middle of pushing the cap 44 into the distal nozzle portion 56, the cap 44 may be inclined with respect to the distal nozzle portion 56 as illustrated in Figs. 4 and 5 due to various factors.

As illustrated in Fig. 4, in a syringe assembly 112A of the comparative example, no ring-shaped protrusion 62 is formed on a sealing surface 160 of a distal nozzle portion 156. In the process of pushing a cap 44, a seal member 46 is in surface contact with a pressing surface 48 of the cap 44 and the sealing surface 160 of the distal nozzle portion 156 to form a pressing space 164. When the cap 44 is inclined in the case of the comparative example, the pressing space 164 has a shape that is open in a V shape so as to gradually expand toward the inner peripheral side as a width D1 in the axial direction on the inner peripheral side becomes larger than a width D2 in the axial direction on the outer peripheral side. Therefore, in the process of pushing the cap 44 to the proximal side, a wall of the seal member 46 moves so as to escape from the pressing space 164 toward a non-pressed site.

As a result, a compressive load on the sealing surface 160 varies in the circumferential direction of the seal member 46, and a pressure limit decreases. That is, in the seal member 46, a wall thickness of a portion that first abuts on the sealing surface 160 is insufficient, and the sealing performance between the sealing surface 160 and the seal member 46 deteriorates in the state after the cap 44 is mounted.

On the other hand, in the syringe assembly 12A of the present embodiment, the ring-shaped protrusion 62 is formed on the inner peripheral portion of the sealing surface 60 of the distal nozzle portion 56 as illustrated in Fig. 5. In the process of pushing the cap 44, the seal member 46 is in surface contact with the pressing surface 48 of the cap 44 and the sealing surface 60 of the distal nozzle portion 56 to form the pressing space 64. Since the ring-shaped protrusion 62 is provided as illustrated in the drawing, the width D1 in the axial direction on the inner peripheral side of the pressing space 64 is smaller than the width D2 in the axial direction on the outer peripheral side thereof, and the width D1 in the axial direction of the inner peripheral portion of the pressing space 64 is the narrowest. As a result, the movement of the wall of the seal member 46 is prevented. As a result, a compressive load on the sealing surface 60 becomes uniform in the circumferential direction of the seal member 46, and the pressure limit can be improved.

Next, a description will be given regarding results obtained by actually preparing the syringe assembly 112A of the comparative example (Experimental Example 1) and the syringe assembly 12A of the present embodiment (Experimental Example 2) and measuring a pressure limit at which liquid leakage occurs when the gasket 26 is pushed.

In Experimental Example 1, five samples in which water was sealed as a test liquid in the syringe assembly 112A of Comparative Example 1 illustrated in Fig. 4 were prepared. Then, a gasket 26 was pushed by a test plunger, and a displacement (mm) of the test plunger and a test force (N), which is an input load with respect to the test plunger, were measured.

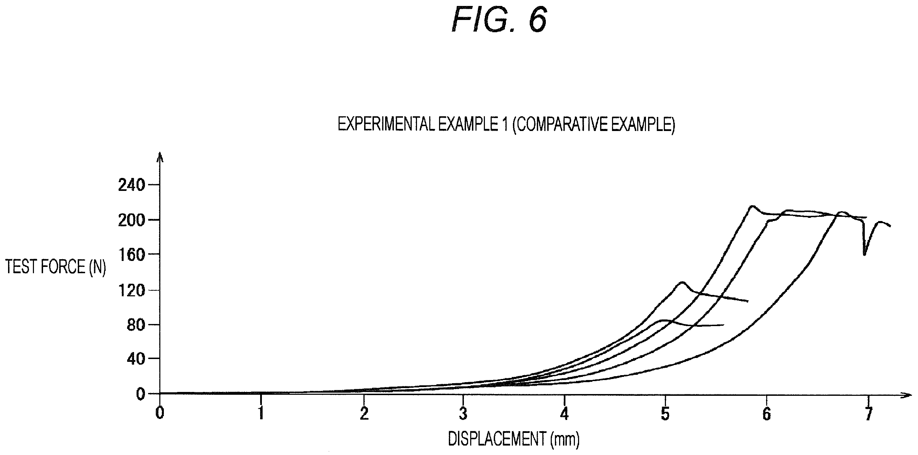

Fig. 6 illustrates measurement results of Experimental Example 1. In Fig. 6, the vertical axis represents the test force (N), and the horizontal axis represents the displacement (mm) of the test plunger. The test force reflects the internal pressure of a syringe 24 and increases as the displacement of the test plunger increases. When liquid leakage occurs, the test force does not increase and becomes constant even if the displacement of the test plunger increases. Therefore, the maximum value of the test force reflects the pressure limit by the seal member 46 in Fig. 6.

As illustrated in the drawing, in Experimental Example 1, the maximum value of the test force ranges from 80 to 200 N with a relatively large variation. Regarding two samples, the pressure limit was an extremely low value between 80 and 120 N.

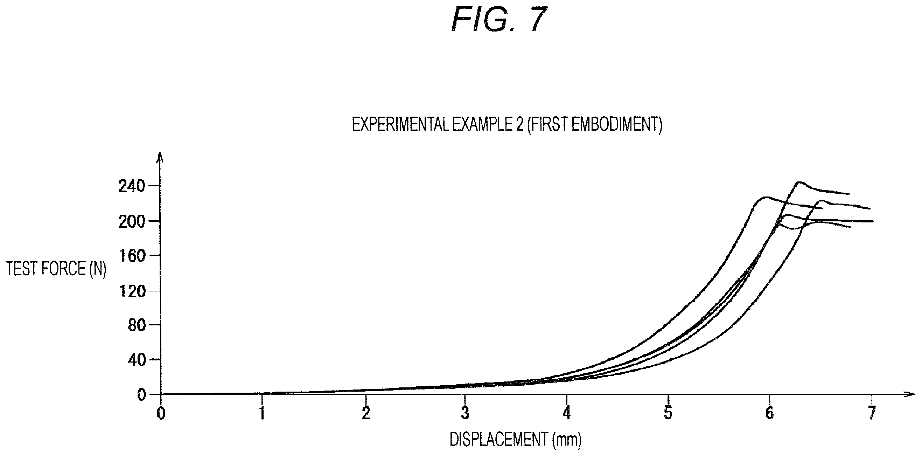

On the other hand, in Experimental Example 2, five samples in which water was sealed in the syringe assembly 12A of the present embodiment illustrated in Fig. 3 were prepared, and the pressure limit was evaluated by the same method as in Experimental Example 1. Fig. 7 illustrates measurement results of Experimental Example 2.

As illustrated in Fig. 7, in Experimental Example 2 (the present embodiment), the maximum value of the test force fell within the range of 200 to 240 N, and it has been confirmed that a variation in the pressure limit was suppressed, and there is no sample having an extremely low pressure limit.

The syringe assembly 12A and the liquid-medicine administration device 10 of the present embodiment have the following effects.

The syringe assembly 12A of the present embodiment includes the syringe 24 having the distal nozzle portion 56, the cap 44 fixed to the distal nozzle portion 56, and the seal member 46 arranged between the cap 44 and the distal nozzle portion 56 and made of the elastic body that seals the distal nozzle portion 56. In the syringe assembly 12A, the cap 44 has the pressing surface 48 that is in surface contact with the distal end of the seal member 46 and presses the seal member 46 to the proximal side, the distal nozzle portion 56 has the sealing surface 60, which opposes the pressing surface 48 and is in surface contact with the proximal end of the seal member 46, at the distal end and the distal opening 58 which is formed on the inner peripheral side of the sealing surface 60 and communicates with the lumen 13 of the syringe 24, the seal member 46 closes the distal opening 58 and is pressed and held in the axial direction in the pressing space 64 over a whole region of the opposing portion between the pressing surface 48 and the sealing surface 60, and the gap between the pressing surface 48 and the sealing surface 60 in the pressing space 64 is narrowest on the inner peripheral side.

According to the above configuration, it is possible to prevent the movement of the wall of the seal member 46 even when the cap 44 is inclined with respect to the distal nozzle portion 56 at the time of mounting the cap 44 on the distal nozzle portion 56. As a result, it is possible to suppress the variation in the compressive load of the seal member 46 in the circumferential direction and to prevent the decrease in the pressure limit caused by the seal member 46.

In the syringe assembly 12A, the sealing surface 60 may be provided with the ring-shaped protrusion 62 which is formed in the annular shape along the inner peripheral side and protrudes toward the pressing surface 48. Since the ring-shaped protrusion 62 is provided on the inner peripheral side in this manner, the gap between the pressing surface 48 and the sealing surface 60 in the pressing space 64 can be made narrowest on the inner peripheral side, and the movement of the wall of the seal member 46 can be prevented.

In addition, the liquid-medicine administration device 10 of the present embodiment includes: the syringe assembly 12A that includes the syringe 24 having the distal nozzle portion 56, the cap 44 fixed to the distal nozzle portion 56; the seal member 46 arranged between the cap 44 and the distal nozzle portion 56 and made of the elastic body that seals the distal nozzle portion 56, and the cap 44 having the pressing surface 48 that is in surface contact with the distal end of the seal member 46 and presses the seal member 46 to the proximal side, the distal nozzle portion 56 having the sealing surface 60, which opposes the pressing surface 48 and is in surface contact with the proximal end of the seal member 46, at the distal end and the distal opening 58 which is formed on the inner peripheral side of the sealing surface 60 and communicates with the lumen 13 of the syringe 24, the seal member 46 closing the distal opening 58 and being pressed and held in the axial direction in the pressing space 64 over the whole region of the opposing portion between the pressing surface 48 and the sealing surface 60, and a gap between the pressing surface 48 and the sealing surface 60 in the pressing space 64 being narrowest on the inner peripheral side; the gasket 26 arranged to be slidable inside the syringe 24; the plunger assembly 14 capable of pressing the gasket 26 in the distal direction; and the drive mechanism 28 driving the plunger assembly 14.

According to the liquid-medicine administration device 10 having the above configuration, the variation in the pressure limit of the seal member 46 is suppressed, and thus, the liquid leakage hardly occurs.

### [Illustrative example not falling under the scope of the claims ]

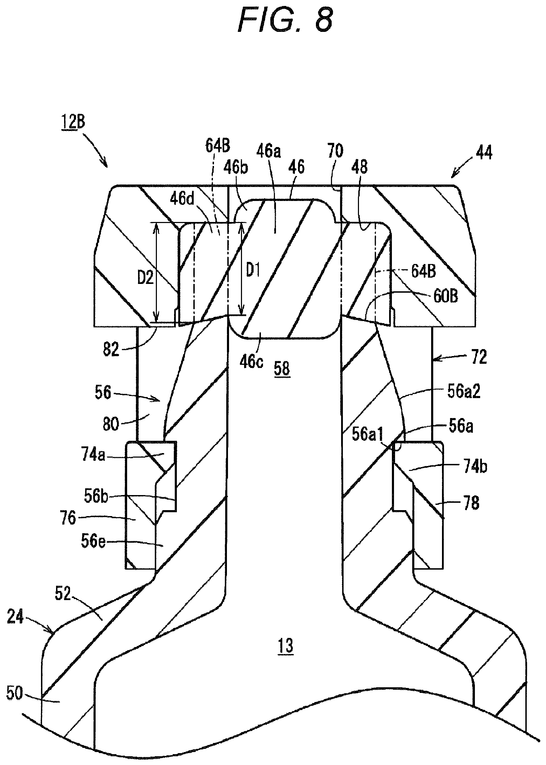

As illustrated in Fig. 8, the syringe assembly 12B of the present embodiment is configured such that a sealing surface 60B of the distal nozzle portion 56 is an inclined surface inclined with respect to the pressing surface 48 of the cap 44. In the sealing surface 60B, the inner peripheral side adjacent to the distal opening 58 protrudes toward the pressing surface 48 the most. That is, the sealing surface 60B is configured as the inclined surface inclined so as to gradually protrude toward the pressing surface 48 from the outer peripheral side to the inner peripheral side. In this manner, a width D1 in the axial direction on the inner peripheral side of a pressing space 64B, which presses the outer peripheral portion 46d of the seal member 46 in the axial direction, is the narrowest even in the syringe assembly 12B.

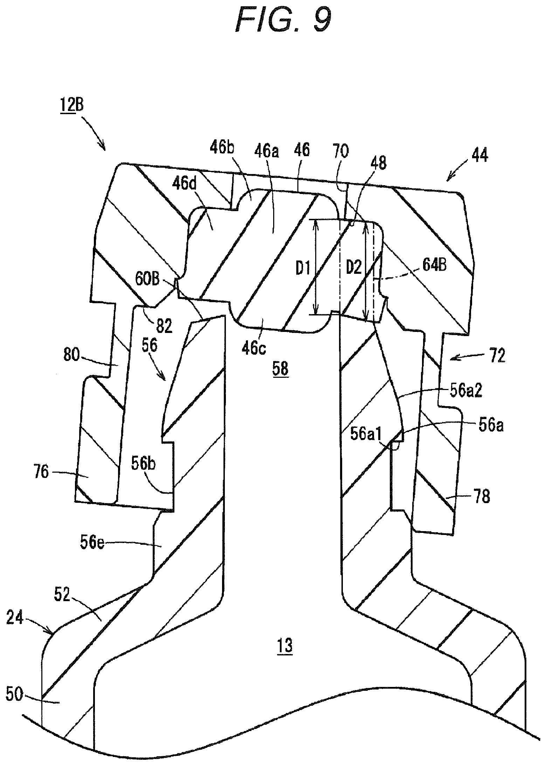

Since the width D1 in the axial direction on the inner peripheral side of the pressing space 64B is the narrowest even when the cap 44 is inclined, movement of a wall of the seal member 46 can be prevented in a cap mounting step as illustrated in Fig. 9. As a result, a pressing load in the circumferential direction of the seal member 46 is made uniform even by the present embodiment so that a variation in a pressure limit can be suppressed.

The syringe assembly 12B of the present embodiment has the following effects.

In the syringe assembly 12B of the present embodiment, the sealing surface 60B is configured as the inclined surface inclined so as to gradually protrude toward the pressing surface from the outer peripheral side to the inner peripheral side. With this configuration, the pressing load in the circumferential direction of the seal member 46 is made uniform so that the variation in the pressure limit can be suppressed.

### [Illustrative example not falling under the scope of the claims I

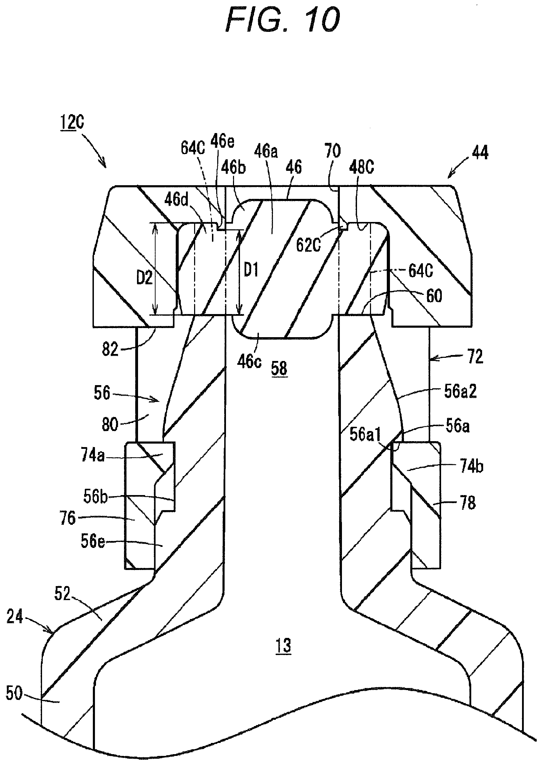

As illustrated in Fig. 10, the syringe assembly 12C of the present embodiment is provided with a ring-shaped protrusion 62C, which protrudes toward the sealing surface 60 of the distal nozzle portion 56, in an inner peripheral portion of a pressing surface 48C of the cap 44. The ring-shaped protrusion 62C is formed in an annular shape along the through-hole 70 of the cap 44. In the seal member 46, a ring-shaped groove 46e is formed in a portion corresponding to the ring-shaped protrusion 62C, and the ring-shaped protrusion 62C is inserted into the ring-shaped groove 46e.

Meanwhile, the sealing surface 60 of the distal nozzle portion 56 is formed as a flat surface perpendicular to the axial direction. Although the ring-shaped protrusion 62 (see Fig. 3) is not formed on the sealing surface 60 in the illustrated example, the present invention is not limited thereto, and the ring-shaped protrusion 62 may be formed on the inner peripheral side of the sealing surface 60.

In the present embodiment, the inner peripheral side where the pressing surface 48C is adjacent to the through-hole 70 protrudes toward the sealing surface 60 the most. Therefore, a width D1 in the axial direction on the inner peripheral side of a pressing space 64C is the narrowest in the pressing space 64C that presses the seal member 46.

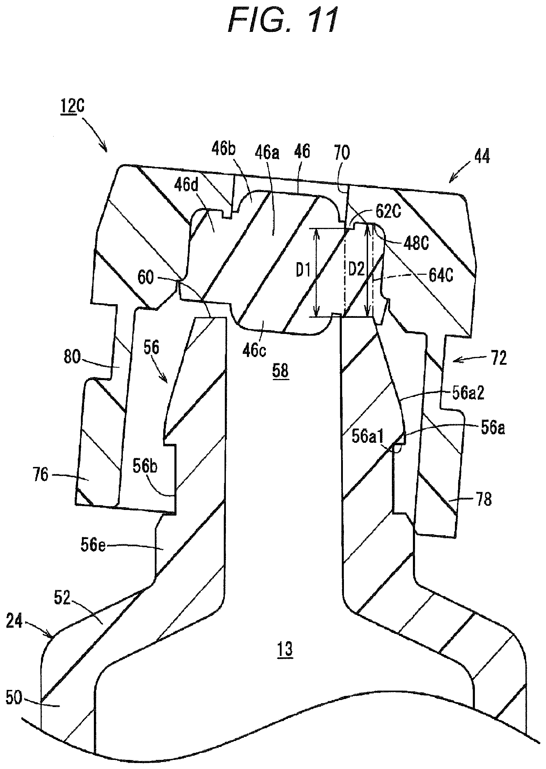

Since the width D1 in the axial direction on the inner peripheral side of the pressing space 64C is the narrowest even when the cap 44 is inclined, movement of a wall of the seal member 46 can be prevented in a cap mounting step as illustrated in Fig. 11. As a result, a pressing load in the circumferential direction of the seal member 46 is made uniform even by the present embodiment so that a variation in a pressure limit can be suppressed.

The syringe assembly 12C of the present embodiment has the following effects.

In the syringe assembly 12C of the present embodiment, the pressing surface 48C is provided with the ring-shaped protrusion 62C which is formed in the annular shape along the inner peripheral side and protrudes toward the sealing surface 60. With this configuration, the pressing load in the circumferential direction of the seal member 46 is made uniform so that the variation in the pressure limit can be suppressed.

### [Illustrative example not falling under the scope of the claims]

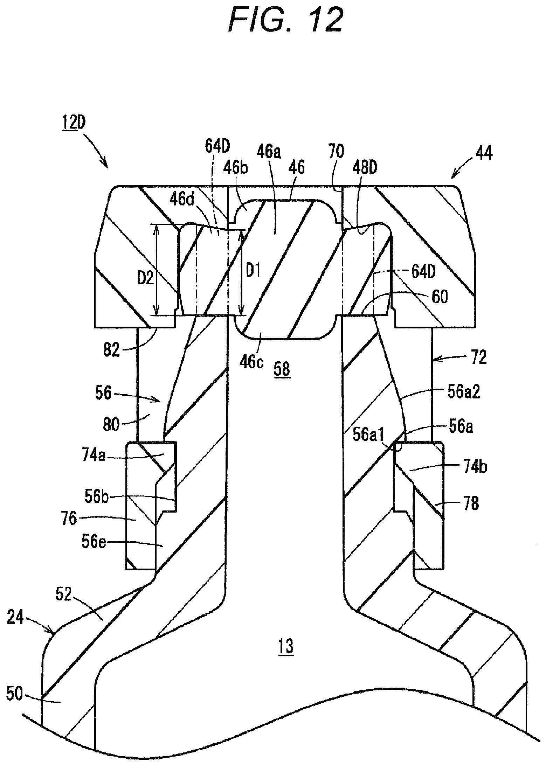

As illustrated in Fig. 12, the syringe assembly 12D of the present embodiment is configured such that a pressing surface 48D of the cap 44 is an inclined surface inclined with respect to the sealing surface 60 of the distal nozzle portion 56. The pressing surface 48D protrudes toward the sealing surface 60 the most on the inner peripheral side adjacent to the through-hole 70. That is, the pressing surface 48D is configured as the inclined surface inclined so as to gradually protrude toward the sealing surface 60 from the outer peripheral side to the inner peripheral side. In addition, an upper end side of the outer peripheral portion 46d of the seal member 46 of the present embodiment is configured as an inclined surface corresponding to the inclination of the pressing surface 48D.

Meanwhile, the sealing surface 60 of the distal nozzle portion 56 is formed as a flat surface perpendicular to the axial direction. Although the ring-shaped protrusion 62 (see Fig. 3) is not formed on the sealing surface 60 in the illustrated example, the present invention is not limited thereto, and the ring-shaped protrusion 62 may be formed on the inner peripheral side of the sealing surface 60.

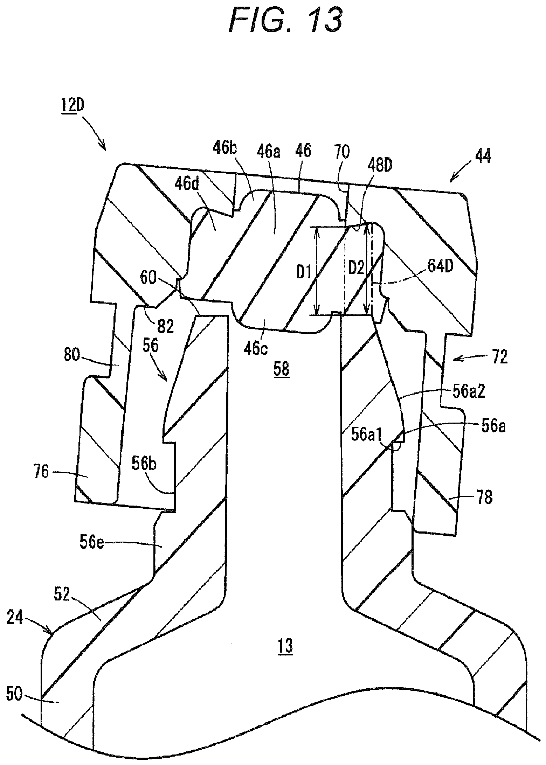

Since a width D1 in the axial direction on the inner peripheral side of the pressing space 64D is the narrowest even when where the cap 44 is inclined, movement of a wall of the seal member 46 can be prevented in a cap mounting step as illustrated in Fig.13. As a result, a pressing load in the circumferential direction of the seal member 46 is made uniform even by the present embodiment so that a variation in a pressure limit can be suppressed.

The syringe assembly 12D of the present embodiment has the following effects.

In the syringe assembly 12D, the pressing surface 48D is configured as the inclined surface inclined so as to gradually protrude toward the sealing surface 60 from the outer peripheral side to the inner peripheral side. With this configuration, the pressing load in the circumferential direction of the seal member 46 is made uniform so that the variation in the pressure limit can be suppressed.

Although the syringe assemblies 12A, 12B, 12C, and 12D, and the liquid-medicine administration device 10 have been described with the preferred embodiments as above, it is obvious that the present invention is not limited to the above-described embodiments, and various modifications can be made within a scope of the present invention.

## Claims

1. A syringe assembly (12A) comprising: a syringe (24) having a distal nozzle portion (56); a cap (44) fixed to the distal nozzle portion (56); and a seal member (46) arranged between the cap (44) and the distal nozzle portion (56) and made of an elastic body that seals the distal nozzle portion (56),
wherein the cap (44) has a pressing surface (48) that is in surface contact with a distal end of the seal member (46) and presses the seal member (46) to a proximal side,
the distal nozzle portion (56) has a sealing surface (60), which opposes the pressing surface (48) and is in surface contact with a proximal end of the seal member (46) at a distal end and a distal opening (58) which is formed on an inner peripheral side of the sealing surface (60) and communicates with a lumen (13) of the syringe (24),
the seal member (46) closes the distal opening (58, and
a pressing space (64) between the pressing surface (48) and the sealing surface (60) is narrowest on an inner peripheral side of the sealing surface (60),
wherein the sealing surface (60) is provided with a ring-shaped protrusion (62) formed in an annular shape along an inner peripheral side of the sealing surface (60) and protruding toward the pressing surface (48),
**characterized in that**
the seal member (46) is pressed and held in an axial direction in a pressing space (64) over a whole region of an opposing portion between the pressing surface (48) and the sealing surface (60).

2. The syringe assembly according to claim 1, wherein the sealing surface is configured as an inclined surface inclined to gradually protrude toward the pressing surface from an outer peripheral side to an inner peripheral side.

3. The syringe assembly according to claim 1, wherein the pressing surface (48) is provided with a ring-shaped protrusion (62C) formed in an annular shape along an inner peripheral side of the cap (44) and protruding toward the sealing surface (60).

4. The syringe assembly according to claim 1, wherein the pressing surface (48) is configured as an inclined surface inclined to gradually protrude toward the sealing surface (60) from an outer peripheral side to an inner peripheral side.

5. A liquid-medicine administration device comprising a syringe assembly (12A) according to claim 1, further including:
a gasket (26) arranged to be slidable inside the syringe;
a plunger assembly (14) capable of pressing the gasket (26) in a distal direction; and
a drive mechanism (28) driving the plunger assembly (14).

## Patentansprüche

1. Spritzenanordnung (12A), umfassend: eine Spritze (24), die einen distalen Düsenabschnitt (56) aufweist; eine Kappe (44), die an dem distalen Düsenabschnitt (56) befestigt ist; und ein Dichtungselement (46), das zwischen der Kappe (44) und dem distalen Düsenabschnitt (56) angeordnet ist und aus einem elastischen Körper hergestellt ist, der den distalen Düsenabschnitt (56) abdichtet,
wobei die Kappe (44) eine Druckfläche (48) aufweist, die in Oberflächenkontakt mit einem distalen Ende des Dichtungselements (46) steht und das Dichtungselement (46) zu einer proximalen Seite drückt,
der distale Düsenabschnitt (56) eine Dichtfläche (60), die der Druckfläche (48) gegenüberliegt und an einem distalen Ende in Oberflächenkontakt mit einem proximalen Ende des Dichtungselements (46) steht, und eine distale Öffnung (58) aufweist, die an einer inneren Umfangsseite der Dichtfläche (60) ausgebildet ist und mit einem Lumen (13) der Spritze (24) in Verbindung steht,
das Dichtungselement (46) die distale Öffnung (58) verschließt, und
ein Druckraum (64) zwischen der Druckfläche (48) und der Dichtungsfläche (60) an einer inneren Umfangsseite der Dichtungsfläche (60) am engsten ist,
wobei die Dichtungsfläche (60) mit einem ringförmigen Vorsprung (62) versehen ist, der in einer ringförmigen Form entlang einer inneren Umfangsseite der Dichtfläche (60) ausgebildet ist und in Richtung zu der Druckfläche (48) hin vorsteht,
**dadurch gekennzeichnet, dass**
das Dichtungselement (46) in axialer Richtung in einem Druckraum (64) über einen gesamten Bereich eines gegenüberliegenden Abschnitts zwischen der Druckfläche (48) und der Dichtfläche (60) gedrückt und gehalten wird.

2. Spritzenanordnung nach Anspruch 1, wobei die Dichtfläche als eine geneigte Fläche ausgebildet ist, die so geneigt ist, dass sie von einer äußeren Umfangsseite zu einer inneren Umfangsseite allmählich in Richtung zu der Druckfläche hin vorsteht.

3. Spritzenanordnung nach Anspruch 1, wobei die Druckfläche (48) mit einem ringförmigen Vorsprung (62C) versehen ist, der in einer ringförmigen Form entlang einer inneren Umfangsseite der Kappe (44) ausgebildet ist und in Richtung zu der Druckfläche (60) hin vorsteht.

4. Spritzenanordnung nach Anspruch 1, wobei die Druckfläche (48) als eine geneigte Fläche ausgebildet ist, die so geneigt ist, dass sie von einer äußeren Umfangsseite zu einer inneren Umfangsseite allmählich in Richtung zu der Dichtfläche (60) hin vorsteht.

5. Vorrichtung zur Verabreichung von flüssigen Medikamenten, die eine Spritzenanordnung (12A) nach Anspruch 1 umfasst, ferner umfassend:
eine Dichtung (26), die so angeordnet ist, dass sie innerhalb der Spritze verschiebbar ist;
eine Kolbenanordnung (14), die in der Lage ist, die Dichtung (26) in eine distale Richtung zu drücken; und einen Antriebsmechanismus (28), der die Kolbenanordnung (14) antreibt.

## Revendications

1. Ensemble seringue (12A) comprenant : une seringue (24) ayant une partie de buse distale (56) ; un capuchon (44) fixé à la partie de buse distale (56) ; et un organe d'étanchéité (46) agencé entre le capuchon (44) et la partie de buse distale (56) et constitué d'un corps élastique qui scelle la partie de buse distale (56),
dans lequel le capuchon (44) présente une surface de pression (48) qui est en contact de surface avec une extrémité distale de l'organe d'étanchéité (46) et presse l'organe d'étanchéité (46) vers un côté proximal,
la partie de buse distale (56) présente une surface d'étanchéité (60), qui s'oppose à la surface de pression (48) et est en contact de surface avec une extrémité proximale de l'organe d'étanchéité (46) à une extrémité distale et une ouverture distale (58) qui est formée sur un côté périphérique intérieur de la surface d'étanchéité (60) et communique avec une lumière (13) de la seringue (24),
l'organe d'étanchéité (46) ferme l'ouverture distale (58), et
un espace de pression (64) entre la surface de pression (48) et la surface d'étanchéité (60) est le plus étroit sur un côté périphérique intérieur de la surface d'étanchéité (60),
dans lequel la surface d'étanchéité (60) est pourvue d'une saillie en forme d'anneau (62) formée en forme annulaire le long d'un côté périphérique intérieur de la surface d'étanchéité (60) et faisant saillie vers la surface de pression (48),
**caractérisé en ce que**
l'organe d'étanchéité (46) est pressé et maintenu dans une direction axiale dans un espace de pression (64) sur toute une région d'une partie opposée entre la surface de pression (48) et la surface d'étanchéité (60).

2. Ensemble seringue selon la revendication 1, dans lequel la surface d'étanchéité est configurée comme une surface inclinée inclinée pour faire progressivement saillie vers la surface de pression d'un côté périphérique extérieur vers un côté périphérique intérieur.

3. Ensemble seringue selon la revendication 1, dans lequel la surface de pression (48) est pourvue d'une saillie en forme d'anneau (62C) formée en forme annulaire le long d'un côté périphérique intérieur du capuchon (44) et faisant saillie vers la surface d'étanchéité (60).

4. Ensemble seringue selon la revendication 1, dans lequel la surface de pression (48) est configurée comme une surface inclinée inclinée pour faire progressivement saillie vers la surface d'étanchéité (60) d'un côté périphérique extérieur à un côté périphérique intérieur.

5. Dispositif d'administration de médicament liquide comprenant
un ensemble seringue (12A) selon la revendication 1, comportant en outre :
un joint (26) agencé pour être coulissant à l'intérieur de la seringue ;
un ensemble piston (14) capable de presser le joint (26) dans une direction distale ; et
un mécanisme d'entraînement (28) entraînant l'ensemble plongeur (14).
